# EUROPEAN PATENT APPLICATION

(11) **EP 2 762 151 A1**
(43) Date of publication of application: **06.08.2014**
(21) Application number: 12837135.8
(22) Date of filing: 31.08.2012
(51) Int. Cl.: A61K 38/46, A23L 1/305, A61K 35/74, A61P 25/18, A61P 39/02, C12N 9/48, C12N 9/58

(54) **EXOGENOUS OPIOID PEPTIDE-DEGRADING ENZYME**

(30) Priority: 29.09.2011 JP 2011215525
(71) Applicant: Amano Enzyme Inc., Nagoya-shi, Aichi 460-8630 (JP)
(72) Inventor: MATSUBARA Hirotaka, Kakamigahara-shi Gifu 509-0109 (JP); IDO Hiroki, Kakamigahara-shi Gifu 509-0109 (JP); KOIKEDA Satoshi, Kakamigahara-shi Gifu 509-0109 (JP)
(74) Representative: Steinecke, Peter
(86) International application number: PCT/JP2012/072117
(87) International publication number: WO 2013/047082

(57) **Abstract**

The present invention is intended to provide a means for efficiently degrades exogenous opioid peptides. Provided is an exogenous opioid peptide-degrading enzyme preparation which contains one or more components selected from the group consisting of enzyme preparations from *Penicillium citrinum, Aspergillus oryzae,* and *Aspergillus melleus,* and exhibits a degradation activity for a wheat gluten-derived opioid peptide and a casein-derived opioid peptide.

## Description

### TECHNICAL FIELD

The present invention relates to an enzyme preparation and the uses thereof. More specifically, the present invention relates to an enzyme preparation showing a degradation activity for exogenous opioid peptides, and pharmaceutical compositions and others. This application claims the benefit of priority from prior Japanese Patent Application No. 2011-215525, filed September 29, 2011, the entire contents of which are incorporated herein by reference.

### BACKGROUND ART

It has been reported that food-derived opioid peptide is involved in the development and psychiatric disorders such as autism and brain function disorders (Sun Z and Cade R. Peptides. 24(2), 321-3 (2003).; Shattock P & Whiteley, P, Expert Opin. Ther. Targets 6 (2),175-183 (2002).; Cade R et al. Nutritional Neurosci 3, 57-72 (2000).; Knivsberg AM et al., Nutritional Neurosci 3, 251-61 (2002).; Muruganandam A et al., FASEB-J 16(2), 240-2 (2002).; Reichelt KL et al., Dev Brain Dysfunction 7, 71-85 (1994).; Shattock P et al., Brain Dysfunction 3, 328-45 (1990).; Sun Z et al., Autism 3 (1), 67-83 (1999).; Christison GW and Ivany K, J Dev Behav Pediatr 27 (2 Suppl), S162-71 (2006)). In particular, casomorphin (milk-derived) and gliadorphin (wheat-derived) are attracting attention as the factors aggravating autism. In addition, it has been suggested that these peptides cannot be digested (degraded) in the child body with childhood autism. Therefore, gluten-free and casein-free diet may be put on the patients with childhood autism. There are attempts to relieve the autism symptoms by the enzyme compositions containing a casomorphin/gluteomorphine inhibitor (Patent Documents 1 to 6)

### PRIOR ART DOCUMENT

### PATENT DOCUMENT

Patent Document 1: U.S. Patent No. 6,899,876
Patent Document 2: U.S. Patent No. 6,821,514
Patent Document 3: U.S. Patent No. 6,808,708
Patent Document 4: U.S. Patent No. 6,447,772
Patent Document 5: U.S. Patent No. 6,251,391
Patent Document 6: U.S. Patent Publication No. 2007/0092501

### SUMMARY OF THE INVENTION

### PROBLEM TO BE SOLVED BY THE INVENTION

The present invention is intended to provide a means for efficiently degrading an exogenous opioid peptide, thereby contributing the establishment of the therapy for diseases and clinical states related to an exogenous opioid peptide.

### MEANS FOR SOLVING PROBLEM

In view of the above-described problems, the present inventors studied the degradation activity of various enzyme preparations for gliadorphin and casomorphin. As a result of dedicated research, they found the enzyme preparations showing strong degradation activity and promoting efficient degradation of gliadorphin and casomorphin. In addition, it was also revealed that the combination of a plurality of enzyme preparations, which may be further combined with a proteolytic enzyme, achieves synergistic effect, and allows highly efficient degradation of gliadorphin and casomorphin. Therefore, the study by the present inventors has found the enzyme preparations which are expected to be effective for the treatment of autism, and has revealed more effective usage (more specifically, specific embodiments of combinations). The following aspects of the present invention are based on these findings.
[1] An exogenous opioid peptide-degrading enzyme preparation containing one or more components selected from the group consisting of an enzyme preparation from *Penicillium citrinum,* an enzyme preparation from *Aspergillus oryzae,* and an enzyme preparation from *Aspergillus melleus,* wherein the exogenous opioid peptide-degrading enzyme preparation exhibits a degradation activity for a wheat gluten-derived opioid peptide and a casein-derived opioid peptide.
[2] The exogenous opioid peptide-degrading enzyme preparation of [1], which contains the above-described three enzyme preparations.
[3] The exogenous opioid peptide-degrading enzyme preparation of [1] or [2], wherein the wheat gluten-derived opioid peptide is a gliadorphin containing the amino acid sequence set forth in SEQ ID NO: 2, and the casein-derived opioid peptide is a casomorphin containing the amino acid sequence set forth in SEQ ID NO: 3.
[4] The exogenous opioid peptide-degrading enzyme preparation of any one of [1] to [3], wherein the enzyme preparation from *Aspergillus melleus* contains a semi-alkaline protease.
[5] A pharmaceutical or food composition for treating an exogenous opioid peptide-related disease, containing the exogenous opioid peptide-degrading enzyme preparation of any one of [1] to [4].
[6] The composition of [5], wherein the exogenous opioid peptide-related disease is autism.
[7] A therapy for an exogenous opioid peptide-related disease, including a step of administering the pharmaceutical composition of [6] in a therapeutically effective amount to a patient with an exogenous opioid peptide-related disease.
[8] The therapy of [7], wherein the exogenous opioid peptide-related disease is autism.
[9] A use of the exogenous opioid peptide-degrading enzyme preparation of any one of [1] to [4] for producing a pharmaceutical or food composition for treating an exogenous opioid peptide-related disease.
[10] The use of [9], wherein the exogenous opioid peptide-related disease is autism.

### BRIEF DESCRIPTION OF THE DRAWINGS

Fig. 1 is a graph showing the degradation activity of the enzyme preparations and four-mixed enzyme preparation for gliadorphin peptide. The evaluation used a gliadorphin determination kit. The ordinate is the residual rate of gliadorphin.
Fig. 2 is a graph showing the degradation activity of the enzyme preparations and four-mixed enzyme preparation for casomorphin peptide. The evaluation used a casomorphin determination kit. The ordinate is the residual rate of casomorphin.
Fig. 3 is the result of analysis of the gliadorphin degradation products. The four-mixed enzyme preparation or the four enzyme/enzyme preparations composing the enzyme preparation were individually allowed to act on gliadorphin. The degradation products thus obtained were subjected to LC-MS.
Fig. 4 is the result of analysis of the casomorphin degradation products. The four-mixed enzyme preparation or the four enzyme/enzyme preparations composing the enzyme preparation were individually allowed to act on casomorphin. The degradation products thus obtained were subjected to LC-MS.
Fig. 5 shows the evaluation of casomorphin degradability and gliadorphin degradability using the FRETS substrate. The four enzyme/enzyme preparations composing the four-mixed enzyme preparation were individually allowed to act on the FRETS substrate containing the amino acid sequence of casomorphin, and the degradation activity was studied. The same evaluation was carried out using the FRETS substrate containing the amino acid sequence of gliadorphin. Left: degradation activity of the casomorphin FRETS substrate, right: degradation activity of the gliadorphin FRETS substrate
Fig. 6 shows the casomorphin and gliadorphin degradation activity in the culture supernatant of the eight strains of *Penicillium citrinum.* Each of the culture supernatants of the eight strains of *Penicillium citrinum* stored in a public culture collection was allowed to act on the casomorphin FRETS substrate and gliadorphin FRETS substrate, and the degradation activity was evaluated.
Fig. 7 shows the result of fluorescence detection of the wheat-derived 33-mer peptide degradation product (a 10-fold concentrated sample was used).
Fig. 8 shows the result of fluorescence detection of the gliadorphin degradation product (a 10-fold concentrated sample was used).
Fig. 9 shows the result of fluorescence detection of the casomorphin degradation product.

### DESCRIPTION OF EMBODIMENTS

### 1. Enzyme preparation

A first aspect of the present invention relates to a novel enzyme preparation. The enzyme preparation of the present invention contains one or more components selected from the group consisting of an enzyme preparation from *Penicillium citrinum,* an enzyme preparation *Aspergillus oryzae,* and an enzyme preparation *Aspergillus melleus.* The enzyme preparation of the present invention shows degradation activity owing to its components for wheat gluten-derived and casein-derived opioid peptides, which are exogenous opioid peptides. In the present description, the term "enzyme preparation" means a composition obtained by processing a material containing the target enzyme (for example, a culture solution of a microorganism), and contains one or more enzymes. The term "processing" herein usually means purification, but the method for the purification is not limited. On the other hand, the term "opioid peptide" means a peptide showing a specific action to an opioid receptor, or its related peptide and may be endogenous or exogenous. Endogenous opioid peptides are broadly divided into endorphins, enkephalins, and dynorphins, depending on the type of the receptor on which they act. The exogenous opioid peptide is also referred to as "exorphin". Typical examples of the exogenous opioid peptide is food-derived opioid peptides. Known examples of the food-derived opioid peptide include β-casomorphin and α-casein-exorphin derived from casein, and gliadorphin (gluteomorphine) derived from wheat gluten (Henschen A. et al., Hoppe-Seyler's Z. Physiol. Chem.,360,1217 (1979).; Lottspeich,F. et al., Hoppe Seyler's Z. Physiol. Chem. 361, 1835-1839 (1980).; Loukas S. et al., Biochemistry. 13, 22 (19): 4567-73 (1983).; Zioudrou C et al., J Biol Chem. 10, 254 (7): 2446-9 (1979).; Fukudome S. et al., FEBS Lett., 296, 107 (1992); Fukudome S. et al., FEBS Lett., 316, 17 (1993).). Gliadorphin is also contained in rye, barley, and oat.

The enzyme preparation of the present invention efficiently degrades wheat gluten-derived and casein-derived opioid peptides. According to one embodiment, the enzyme preparation of the present invention cuts the wheat gluten-derived and casein-derived opioid peptides at one or more positions. On the other hand, as shown in the below-described examples, the enzyme preparation of the present invention showed a strong degradation activity for the amino acid sequence YPQPQPF (Tyr-Pro-Gln-Pro-Gly-Pro-Phe) (SEQ ID NO: 2) which is characteristic to gliadorphin 7, and the amino acid sequence YPFPGPI (Tyr-Pro-Phe-Pro-Gly-Pro-Ile) (SEQ ID NO: 3) which is characteristic to β-casomorphin 7. Accordingly, the enzyme preparation of the present invention is further characterized in that it shows degradation activity for the amino acid sequence YPQPQPF (SEQ ID NO: 2), and/or shows strong degradation activity for the amino acid sequence YPFPGPI (SEQ ID NO: 3). The above-listed Patent Documents 1 to 7 mention the use of the gliadorphin (gluteomorphine) inhibitor, but the sequence of gliadorphin therein is GYYPT (Gly-Tyr-Tyr-Pro-Thr) (SEQ ID NO: 4), GFFP (Gly-Phe-Phe-Pro) (SEQ ID NO: 5), FGGYL (Phe-Gly-Gly-Tyr-Leu) (SEQ ID NO: 6), or FGGY (Phe-Gly-Gly-Tyr) (SEQ ID NO: 7), and is completely different from the sequence of gliadorphin in the present invention (SEQ ID NO: 2). At least at this point, the present invention should be distinguished from the arts disclosed in Patent Documents 1 to 7.

As shown in the below-described examples, in particular, each of the enzyme preparations from *Penicillium citrinum, Aspergillus oryzae,* and *Aspergillus melleus* showed strong degradation activity for gliadorphin and casomorphin. Among them, the enzyme preparation from *Penicillium citrinum* showed the strongest degradation activity. In consideration of this fact, it is preferred that the enzyme preparation from *Penicillium citrinum* be used as a single component or a component to be combined. Therefore, one embodiment of the present invention contains the enzyme preparation from *Penicillium citrinum* as an essential component. Another embodiment contains the enzyme preparation from *Penicillium citrinum* as a first component, and the enzyme preparation from *Aspergillus oryzae* as a second component. Yet another embodiment contains the enzyme preparation from *Aspergillus melleus* as a third component. The combination of the enzyme preparations from *Penicillium citrinum, Aspergillus oryzae,* and *Aspergillus melleus* is expected to achieve additive or synergistic effect, as shown in the examples.

Gliadorphin and casomorphin have a homologous amino acid sequence motif "Tyr-Pro-X-Pro-X-Pro-X (SEQ ID NO: 8)", and are praline-rich resistant peptides. The enzyme preparation from *Penicillium citrinum* exhibits a high degradation activity for gliadorphin and casomorphin as shown in the examples. In other words, it exhibits a degradation activity which can be detected by the FRETS method shown in the below-described examples using the FRETS substrate containing the both peptide sequences (degradability evaluation by the FRETS substrate). The enzymatic activity liberating 1 µM of Nma per minute upon reaction at 37°C is set as 1 unit (u).

The degradation activity of the enzyme preparation from *Penicillium citrinum* was evaluated by the above-described method, and an excellent degradation activity for gliadorphin (SEQ ID NO: 2) (about 6,300 u/g) and casomorphin (SEQ ID NO: 3) (about 5,900 u/g) was detected.

The enzyme preparation from *Aspergillus oryzae* also exhibited a high degradation activity for gliadorphin (SEQ ID NO: 2) (about 6,000 u/g) and casomorphin (SEQ ID NO: 3) (about 2,200 u/g). In addition, the enzyme preparation from *Aspergillus melleus* also exhibited a strong degradation activity for gliadorphin (SEQ ID NO: 2) (about 14,000 u/g).

The enzyme preparation from *Penicillium citrinum* can be prepared by an ordinary procedure. For example, after filtrating a culture solution of *Penicillium citrinum,* the filtrate is concentrated by, for example, ultrafiltration. As necessary, purification by ammonium sulfate precipitation, dialysis, desalt, or any chromatography is carried out. Furthermore, for example, spray drying or ethanol precipitation may be used to prepare the final product. The enzyme preparation from *Penicillium citrinum* may be used at a predetermined concentration, and may be diluted or concentrated before use.

The enzyme preparation from *Penicillium citrinum* may be replaced with an enzyme preparation derived from a plant, animal, or microorganism, as long as it exhibits a degradation activity equivalent to that of the enzyme preparation from *Penicillium citrinum.* Alternatively, an enzyme preparation derived from other fungi (for example, *Aspergillus niger*) or bacterium (Actinomyces bacterium such as *Streptomyces aureus*) may be used.

The enzyme preparation from *Aspergillus oryzae* may be prepared by an ordinary procedure. For example, a culture solution of *Aspergillus oryzae* is filtered, and then the filtrate is concentrated by, for example, ultrafiltration. As necessary, purification is carried out by ammonium sulfate precipitation, dialysis, desalting, or any chromatography. Furthermore, for example, spray-dry method or ethanol precipitation may be used to obtain the final product. The enzyme preparation from *Aspergillus oryzae* may be used at a predetermined concentration, and may be diluted or concentrated before use.

The enzyme preparation from *Aspergillus melleus* may be prepared by an ordinary procedure. For example, a culture solution of *Aspergillus melleus* is filtered, and then the filtrate is concentrated by, for example, ultrafiltration. As necessary, purification is carried out by ammonium sulfate precipitation, dialysis, desalting, or any chromatography. Furthermore, for example, spray drying or ethanol precipitation may be used to obtain the final product. The enzyme preparation from *Aspergillus melleus* may be used at a predetermined concentration, and may be diluted or concentrated before use.

According to one embodiment, the enzyme preparation from *Aspergillus oryzae* contains at least Oryzin, neutral protease I (NPI), and neutral protease II (NPII).

According to one embodiment, the enzyme preparation from *Aspergillus melleus* contains a semi-alkaline protease. The term "semi-alkaline protease" means an enzyme hydrolyzing a protein and its degradation products (polypeptide, peptide), and exhibits an activity in the pH range of 6.0 to 11.

As an active ingredient of the enzyme preparation of the present invention, a specific protease component contained in the enzyme preparation from *Aspergillus oryzae* may be used. For example, aorsin is a serine proteinase having trypsin-like specificity in the acidic pH range, and can be purified from the enzyme preparation from *Aspergillus oryzae.* The details about aorsin A and aorsin B are described in Japanese Patent No. 4401555 and Japanese Unexamined Patent Application Publication No. 2009-232835. The entire contents of these patent documents are incorporated herein by reference. Aorsin efficiently hydrolyzes a gluten-derived peptide in the acidic pH range. The nucleotide sequence and amino acid sequence of aorsin are disclosed in the literature by Lee et al. (Biockem. J. 371 (PT 2), 541-548 (2003)). The nucleotide sequence of aorsin A is registered in GenBank under accession number AB084899.1. The corresponding amino acid sequence is registered under accession number BAB97387. For aorsin B, the nucleotide sequence and amino acid sequence are registered in GenBank under accession numbers XM001820783.1 and XP_001820835.1, respectively.

An enzyme containing the amino acid sequence of aorsin or an enzyme containing the amino acid sequence having an identity of 70% or more to the amino acid sequence of aorsin may be used. The identity is preferably at least 80%, more preferably at least 90%, and even more preferably at least 99%.

The enzyme having an amino acid sequence equivalent to the amino acid sequence of aorsin may be used. These two amino acid sequences are regarded as equivalent when the identity is at least 80%, preferably at least 90%, and even more preferably at least 99%, and the functions are also equivalent. The equivalent sequences can be identified by, for example, the GAP program of the GCG software package, or the sequence comparison algorithm such as BLAST or FASTA.

According to one embodiment of the present invention, other protease and peptidase, for example, a semi-alkaline protease, a protease (for example, papain, chymopapain, trypsin, or chymotrypsin), or carboxypeptidase may be used as an additional component (optional component). The enzyme preparation of the present embodiment achieves additive or synergistic effect, and allows more efficient degradation of exogenous opioid peptides. Actually, the combination of the enzyme preparations from *Penicillium citrinum, Aspergillus oryzae,* and *Aspergillus melleus* with papain exhibited a very strong degradation activity for the gliadorphin 7-mer peptide (see the below-described example). These components may be combined with, for example, a protease (for example, pepsin, trypsin, or semi-alkaline protease) or a peptidase (for example, carboxypeptidase).

The protease may be a commercial product (for example, Papain W-40). The commercial product is used at the original concentration or after diluted or concentrated. Alternatively, the enzyme may be not a commercial product but a freshly purified protease. The purification method may be, for example, salting out, filtration, ultrafiltration, centrifugation, or any chromatography (for example, ion exchange chromatography, or affinity chromatography).

An activated protease (for example, activated papain) may be used. For example, papain can be activated by a reducing agent. Examples of the reducing agent used for this activation include glutathione, dithiothreitol (DTT), L-cysteine, and N-acetyl L-cysteine.

A protease or peptidase which does not have degradation activity for exomorphin peptide by itself may be used. As shown in the examples, papain did not exhibit direct degradability for exomorphine peptide, but contributes to the improvement in degradability of the gliadorphin-containing peptide.

An endopeptidase and an exopeptidase may be used for degradation. Examples of the exopeptidase include carboxypeptidase Y (CPY). CPY is also referred to as protease C or yscY. CPY is an exopeptidase having a wide range of specificity, and liberates amino acid from the carboxy terminal of a protein or peptide. CPY belongs to the serine carboxypeptidase family, and shows a high level of sequence conservation. The activating region of CPY is surrounded by serine and a histidine residue which are essential for activation. For example, CPY derived from a yeast or fungi (for example, *Saccharomyces, Schizosaccharomyces, Aspergillus, Candida, Pichia*) may be used. More specifically, for example, CPY having an amino acid sequence identity of at least 70%, preferably at least 80%, more preferably at least 90%, and even more preferably 100% with the CPY from *Saccharomyces cerevisiae, Aspergillus niger, Schizosaccharomyces pombe,* or *Aspergillus fumigatus* may be used.

The components of the enzyme preparation of the present invention (i.e. enzyme preparation, enzyme) may be prepared from a natural microorganism which produces these components. Alternatively, these components may be prepared from a transgenic microorganism (so-called mutant).

The components of the enzyme preparation of the present invention (i.e. enzyme preparation, enzyme) may be prepared from a natural plant which produces these components in the form of an enzyme having a degradation activity. Alternatively, these components may be prepared from a transgenic plant (so-called mutant).

Some specific examples of the type and combination of the components contained in the enzyme preparation of the present invention (Examples 1 to 6 of the enzyme preparation) are described below.
Example 1 of enzyme preparation : Contains the enzyme preparation from *Penicillium citrinum* as an essential component
Example 2 of enzyme preparation : Contains the enzyme preparation from *Aspergillus oryzae* as an essential component
Example 3 of enzyme preparation : Contains the enzyme preparation from *Aspergillus melleus* as an essential component
Example 4 of enzyme preparation : Contains the enzyme preparations from *Penicillium citrinum* and *Aspergillus oryzae* as essential components
Example 5 of enzyme preparation : Contains the enzyme preparations from *Aspergillus oryzae, Penicillium citrinum,* and *Aspergillus oryzae* as essential components
Example 6 of enzyme preparation : Contains the enzyme preparations from *Aspergillus oryzae, Penicillium citrinum,* and *Aspergillus oryzae,* and papain as essential components
Example 7 of enzyme preparation : Contains the enzyme preparations from *Aspergillus oryzae* and *Penicillium citrinum,* and papain as essential components

The enzyme preparation of the present invention may further contain, in addition to the active ingredient, for example, an excipient, a buffering agent, a suspending agent, a stabilizer, a preservative, an antiseptic, and a normal saline solution. Examples of the excipient include lactose, sorbitol, D-mannitol, and white sugar. Examples of the buffering agent include phosphates, citrates, and acetates. Examples of the stabilizer include propylene glycol and ascorbic acid. Examples of the preservative include phenol, benzalkonium chloride, benzyl alcohol, chlorobutanol, and methylparaben. Examples of the antiseptic include benzalkonium chloride, paraoxybenzoic acid, and chlorobutanol.

### 2. Pharmaceutical composition, food composition

The enzyme preparation of the present invention exhibits degradation action on exogenous opioid peptides, and may be applied to a disease or clinical state related to an exogenous opioid peptide. Accordingly, another aspect of the present invention provides a pharmaceutical or food composition including the enzyme preparation of the present invention for a disease or clinical state related to an exogenous opioid peptide.

In the present description, the "disease or clinical state related to an exogenous opioid peptide" is referred to as "exogenous opioid peptide-related disease" for convenience. "Related to an exogenous opioid peptide" means that the exogenous opioid peptide is at least a cause of the expression (more specifically development or onset) or progress of the disease or clinical state.

The therapeutic effects include the relief (alleviation) of the symptoms characteristic to or associated with the exogenous opioid peptide-related disease, and prevention or delay of the symptoms. The latter can be regarded as one of the prophylactic effects in the prevention of progression of the disease. In this manner, the therapeutic and prophylactic effects are partially overlapped. Therefore, it is difficult and not beneficial to clearly discriminate them. A typical prophylactic effect is the prevention or delay of the expression (development) of symptoms characteristic to exogenous opioid peptide-related diseases. As long as it shows any therapeutic effect and/or prophylactic effect for exogenous opioid peptide-related diseases, it is classified as a remedy for exogenous opioid peptide-related diseases.

Casomorphin and gliadorphin, which are exogenous opioid peptides, are suggested to be related to autism which is a typical pervasive developmental disorder, and mental disorders (mental sickness) such as integration disorder syndrome, and depression (especially postpartum depression) (Sun Z and Cade R. Peptides. 24 (2), 321-3 (2003).; Shattock P & Whiteley, P, Expert Opin. Ther. Targets 6 (2),175-183 (2002); Cade R et al. Nutritional Neurosci 3, 57-72 (2000).; Knivsberg AM et al., Nutritional Neurosci 3, 251-61 (2002).; Muruganandam A et al., FASEB-J 16 (2), 240-2 (2002).; Reichelt KL et al., Dev Brain Dysfunction 7, 71-85 (1994).; Shattock P et al., Brain Dysfunction 3, 328-45 (1990).; Sun Z et al., Autism 3 (1), 67-83 (1999).; Christison GW and Ivany K, J Dev Behav Pediatr 27 (2 Suppl), S162-71 (2006)). Based on this fact, the "exogenous opioid peptide-related disease" to which the composition of the present invention may be used is, for example, pervasive developmental disorder, integration disorder syndrome, or depression. The pervasive developmental disorder herein includes autism, Asperger's syndrome, Rett's disorder, childhood disintegrative disorder, and pervasive developmental disorder - not otherwise specified. Preferably, the composition of the present invention is provided for the treatment of prevention of autism, integration disorder syndrome, or depression (postpartum depression). More preferably, the composition of the present invention is provided for the treatment or prevention of autism.

The formulation of the pharmaceutical composition of the present invention may use a common procedure. When formulated, other components which are acceptable for formulation (for example, a carrier, an excipient, a disintegrating agent, a buffering agent, an emulsifying agent, a suspending agent, a soothing agent, a stabilizer, a preservative, an antiseptic, and a normal saline solution) may be added. Examples of the excipient include lactose, starch, sorbitol, D-mannitol, and white sugar. Examples of the disintegrating agent include starch, carboxymethyl cellulose, and calcium carbonate. Examples of the buffering agent include phosphates, citrates, and acetates. Examples of the emulsifying agent include gum arabic, sodium alginate, and tragacanth. Examples of the suspending agent include glycerol monostearate, monostearic acid aluminum, methyl cellulose, carboxymethyl cellulose, hydroxymethyl cellulose, and sodium lauryl sulfate. Examples of the soothing agent include benzyl alcohol, chlorobutanol, and sorbitol. Examples of the stabilizer include propylene glycol, and ascorbic acid. Examples of the preservative include phenol, benzalkonium chloride, benzyl alcohol, chlorobutanol, and methylparaben. Examples of the antiseptic include benzalkonium chloride, paraoxybenzoic acid, and chlorobutanol.

The form of formulation is not particularly limited, and the medicine of the present invention may be provided in the form of, for example, pellets, a powder, fine pellets, granules, capsules, a syrup, or an injection.

The pharmaceutical composition of the present invention contains an active ingredient in an amount enough for achieving the expected therapeutic effect (includes prophylactic effect), more specifically, the therapeutic effect for exogenous opioid peptide-related diseases (more specifically, therapeutically effective amount). The amount of the active ingredient in the pharmaceutical composition of the present invention generally depends on the dosage form, and is, for example, about 0.1% to 95% by weight, thereby achieving the intended dose.

The pharmaceutical composition of the present invention is administered to the patient (or potential patient) orally or parenterally according to the dosage form. The method of "parenteral" administration herein is not particularly limited, and examples include intravenous, intramuscular, intraarterial, intraspinal, intracapsular, intraorbital, intracardiac, intradermal, intraperitoneal, percutaneous transluminal, hypodermic, subepidermal, intraarticular, intraarticular, subarachnoid, intraspinal, and intrasternal infusion and injection.

The dose of the pharmaceutical composition of the present invention is established so as to achieve the expected therapeutic effect. For the establishment of the therapeutically effective dose, in general, the symptoms, the age, sex, and body weight of the patient, and other factors are taken into consideration. Those skilled in the art can establish an appropriate dose in consideration of these factors. For example, the dose for an adult (body weight: about 60 kg) may be established in such a manner that the amount of the active ingredient is about 10 mg to 2,000 mg, preferably about 50 mg to 1,000 mg, and more preferably about 100 mg to 700 mg a day. The administration schedule may be, for example, once to several times a day, once every two days, or once every three days. For the making of the administration schedule, the disease state of the patient, and the expected duration of the effect of the active ingredient may be taken into consideration.

As described above, one embodiment of the present invention is a food composition containing the enzyme preparation of the present invention. Examples of the "food composition" of the present invention include general foods (for example, grains, vegetables, meat, various processed foods, confectioneries, milk, refreshing beverages, and alcohol beverages), dietary supplements (supplements and nutrition drinks), and food additives. When used as a dietary supplement or food additive, the form may be, for example, a powder, granules, tablets, a paste, or a liquid. The provision of the enzyme preparation of the present invention in the form of a food composition makes it easy to take the enzyme preparation of the present invention routinely and continuously.

The food composition of the present invention preferably contains an active ingredient in an amount enough for achieving therapeutic or prophylactic effect. The loading may be established in consideration of, for example, the disease state, health condition, age, sex, and body weight of the subject.

### EXAMPLES

### 1. Determination of gliadorphin in the degradation product using gliadorphin determination kit

A 33-mer peptide (LQLQPFPQPQLPYPQPQLPYPQPQLPYPQPQPF: SEQ ID NO: 1), which is a resistant peptide containing gliadorphin, is the amino acid sequence of No. 56-88 of α2 gliadin composing gluten. Using the 33-mer peptide as the substrate, degradation was attempted using various enzymes (pepsin, the enzyme preparation from *Aspergillus oryzae* (AOEP), papain (Asahi Food & Healthcare Co., Ltd.), the enzyme preparation from *Aspergillus melleus* (AMEP), the enzyme preparation from *Penicillium citrinum* (PCEP)), and a four-mixed enzyme preparation (containing AOEP, papain, AMEP, and PCEP). The composition of the reaction liquid and reaction conditions are as follows.
(1) The following solutions are mixed. As controls, a blank prepared by adding miliQ (registered trademark) water in place of an enzyme, and a sample blank prepared by adding an enzyme solution which had been boiled for 10 minutes for heat inactivation were prepared.

| | |
|---|---|
| 33-mer peptide solution (1 mg/mL miliQ (registered trademark)) | 10 µl |
| Enzyme solution (containing 0.2 mg/mL of 20 mM sodium acetate buffer (pH 4.5) and 0.15 mg of an enzyme; when a four-mixed enzyme preparation is used, containing 0.015 mg of ACEP, 0.015 mg of papain, 0.015 mg of AMEP, and 0.015 mg ofPCEP) | 10 µl |
| 150 mM sodium acetate buffer (pH 4.5) | 10 µl |

(2) Allowed to react at 37°C for 1 hour.
(3) Treated at 90°C for 5 minutes to terminate the reaction.
(4) The reaction liquid was cooled to room temperature, diluted as appropriate, and gliadorphin in all the reacted samples was determined using "Gliadorphin-7, EIA Kit, High Sensitivity Cat. No. S-1399" manufactured by BACHEM.

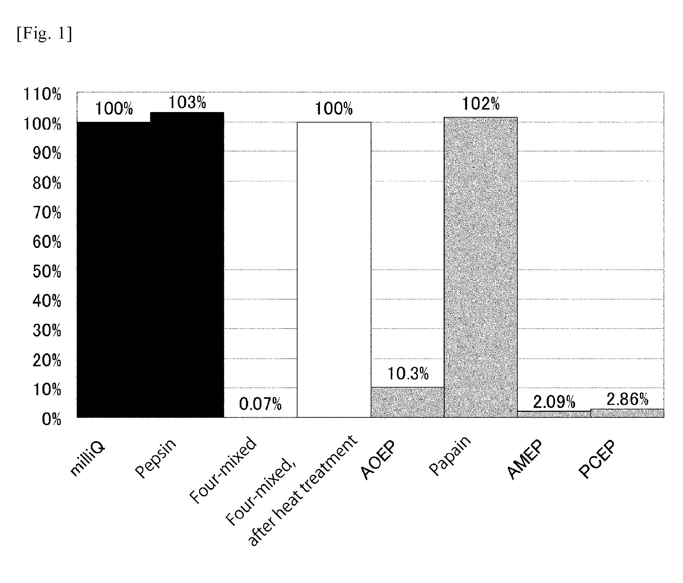

The determination results are shown in Fig. 1. The ordinate of the graph in Fig. 1 is the residual rate of gliadorphin. It is shown that gliadorphin was almost completely degraded by the four-mixed enzyme preparation. In addition, each of AOEP, AMEP, and PCEP exhibited a high degradation activity by itself.

### 2. Determination of casomorphin in the degradation product by casomorphin determination kit

The casomorphin degradability of the enzymes and four-mixed enzyme preparation was studied in the same manner as in 1. The substrate was a resistant peptide containing casomorphin (YPFPGPI: SEQ ID NO: 3). The determination of casomorphin used "Beta Casomorphin, EIA Kit Cat. No. S-1334" manufactured by BACHEM.

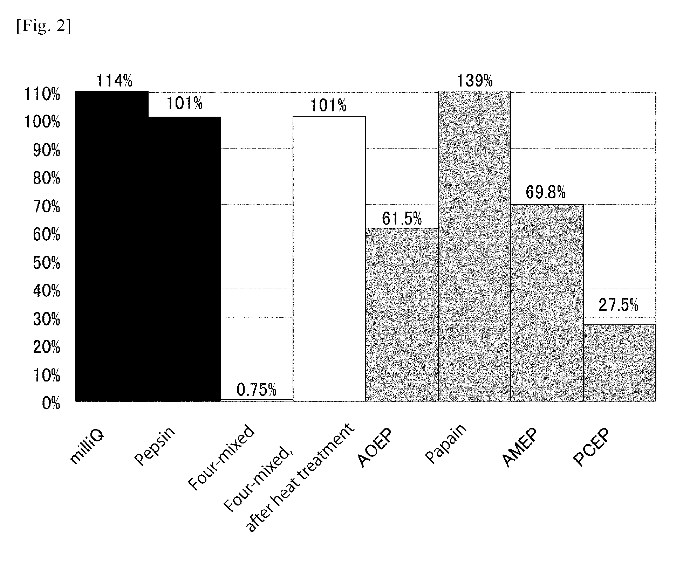

The determination results are shown in Fig. 2. The ordinate of the graph in Fig. 2 is the residual rate of casomorphin. It is shown that casomorphin was almost completely degraded by the four-mixed enzyme preparation. In addition, each of AOEP, AMEP, and PCEP showed a degradation activity by itself. In particular, the degradation activity of PCEP was high.

### 3. Analysis of gliadorphin degradation product by LC-MS

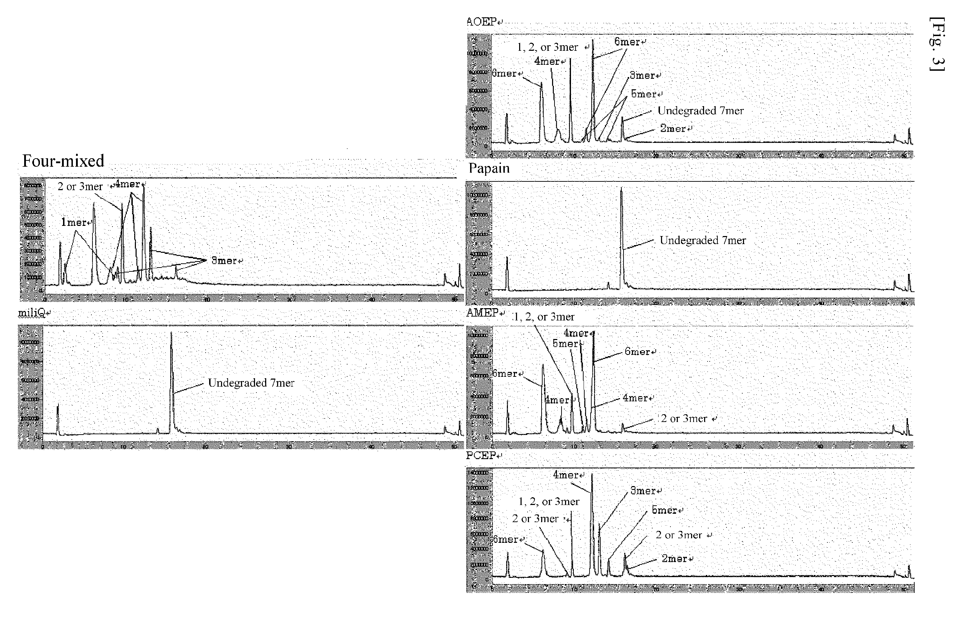

The peptide sequence (YPQPQPF: SEQ ID NO: 2) of gliadorphin was completely synthesized, and used as the substrate. The above-described four-mixed enzyme preparation and each of the four enzyme/enzyme preparation composing it were individually allowed to act on gliadorphin, and the degradation product was analyzed by LC-MS. The sample reacted without enzyme addition was used as the control. In addition, for comparison, the degradation product obtained by the reaction of pepsin was also analyzed. As a result of this, the degradation fragment pattern shown in Fig. 3 was obtained. It is shown that each of PCEP, AOEP, and AMEP degraded gliadorphin by itself. However, an undegraded 7-mer was detected when AOEP and papain were used singly, and the maximum fragment was a 6-mer when AMEP and PCEP were used singly. On the other hand, no undegraded 7-mer was detected when the four-mixed enzyme preparation was used (only a 3-mer was confirmed in the detailed analysis of the peak at the position), and the maximum fragment was a 4-mer. Therefore, the four-mixed enzyme preparation exhibited a synergistic effect.

### 4. Analysis of casomorphin degradation product by LC-MS

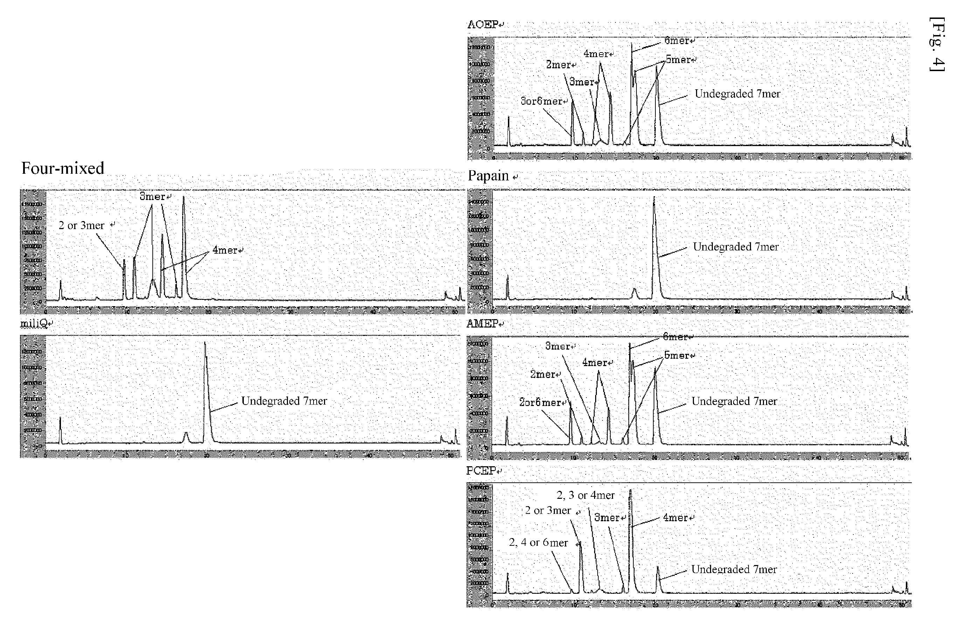

The peptide sequence (YPFPGPI: SEQ ID NO: 3) of casomorphin was completely synthesized, and used as the substrate. The above-described four-mixed enzyme preparation and each of the four enzyme/enzyme preparation composing it were individually allowed to act on casomorphin, and the degradation product was analyzed by LC-MS. The sample reacted without enzyme addition was used as the control. In addition, for comparison, the degradation product obtained by the reaction of pepsin was also analyzed. As a result of this, the degradation fragment pattern shown in Fig. 4 was obtained. It is shown that each of PCEP, AOEP, and AMEP degraded casomorphin by itself. However, undegraded 7-mer was detected when the enzyme/enzyme preparation were used singly, but no undegraded 7-mer was detected when the four-mixed enzyme preparation was used. Therefore, the four-mixed enzyme preparation showed synergistic effect.

### 5. Evaluation of casomorphin and gliadorphin degradability using FRETS substrate

FRETS (Fluorescence Resonance Energy Transfer Substrate) is a protease substrate using a fluorescence group and a quenching group, and used for finding the peptidase which cuts a specific peptide sequence. Using the FRETS, the casomorphin degradation activity of the enzyme/enzyme preparation was evaluated. The evaluation method are described below.
(1) The FRETS substrate containing the amino acid sequence (YPFPGPI: SEQ ID NO: 3) of casomorphin was dissolved in a 20 mM sodium acetate buffer (pH 4.5) at a concentration of 10 µM, and used as the substrate solution.
(2) Each of the enzyme/enzyme preparation was dissolved in a 20 mM sodium acetate buffer (pH 4.5) at a concentration of 0.01% (w/v), and used as the enzyme solution.
(3) 10 µl of the enzyme solution was added to 100 µl of the substrate solution, immediately stirred, and subjected to kinetic measurement for 20 minutes. The excitation light wavelength and fluorescence wavelength were λex (excitation light wavelength) = 340 nm, λem (fluorescence wavelength) = 440 nm. The blank used a 20 mM sodium acetate buffer (pH 4.5) in place of the enzyme solution.
(4) The enzymatic activity was calculated from the fluorescence intensity thus obtained and the value of the calibration curve. The amount of enzyme liberating 1 µmol of Nma at 37°C was defined as 1 unit (u).

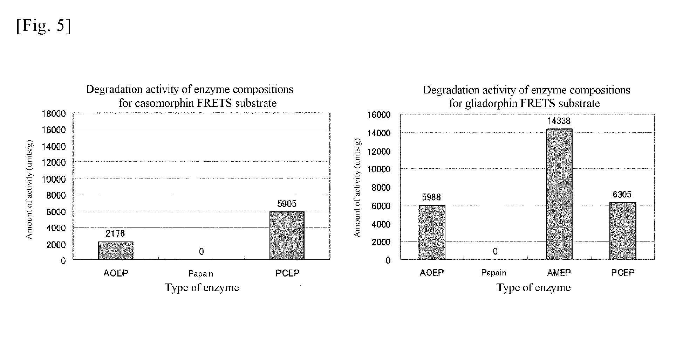

The evaluation results are shown in Fig. 5. In addition, the result of the evaluation using the FRETS substrate containing the amino acid sequence (YPQPQPF: SEQ ID NO: 2) of gliadorphin is also shown. AOEP and PCEP showed a strong casomorphin degradation activity. The degradation activity of PCEP was particularly strong. For gliadorphin, AOEP, AMEP, and PCEP showed a strong degradation activity. The degradation activity of AMEP was markedly strong.

### 6. Degradation of gliadorphin and casomorphin by the culture of Penicillium citrinum stock strains (type culture)

The gliadorphin degradation activity and casomorphin degradation activity of the enzyme preparation from *Penicillium citrinum* (PCEP) were measured by the following method.

Eight strains of *Penicillium citrinum* stored in a public culture collection (NBRC 6026, JCM 22500, JCM 5591 (NBRC 6026), IFO 6225, JCM 22508, JCM 22511, JCM 22517, and JCM 22519) were inoculated from ampoules on Malt extract Agar slant culture media. They were cultured in a thermostat bath at 25°C for 3 to 5 days. The colonies were aseptically cut into a 5 mm square using a platinum wire loop, and inoculated in 50 mL of Malt extract culture medium in a 300-mL conical flask. The colonies were cultured on a rotary (200 rpm, 25°C) for 12 days, and the degradation activity of the FRETS substrates (the FRETS substrate containing the amino acid sequence of gliadorphin or casomorphin) in the culture supernatant was measured. The measurement method followed the method of 5.

### <Constitution and preparation of culture medium>

### (1) Malt extract Agar slant culture medium

| | |
|---|---|
| Malt extract (Difco) | 2% (w/v) |
| D-glucose | 2% (w/v) |
| Bacto Peptone (Difco) | 1% (w/v) |
| Agar | 1.5% (w/v) |

After adjusting the pH to 6.0 and diluting to the mark, the mixture was warmed until the agar dissolved, and dispensed in 7 mL portions into a 18 mm diameter test tube. After pasteurization at 121°C for 20 minutes, the test tube was tilted and allowed to stand overnight, whereby the medium was solidified and used as a slant culture medium.

### (2) Malt extract culture medium

| | |
|---|---|
| Malt extract (Difco) | 2% (w/v) |
| D-glucose | 2% (w/v) |
| Bacto Peptone (Difco) | 1% (w/v) |

After adjusting the pH to 6.0 and diluting to the mark, the mixture was dispensed in 50 mL portions into a 300-mL conical flask. After pasteurization at 121°C for 20 minutes, the medium was cooled to room temperature, and used for culture.

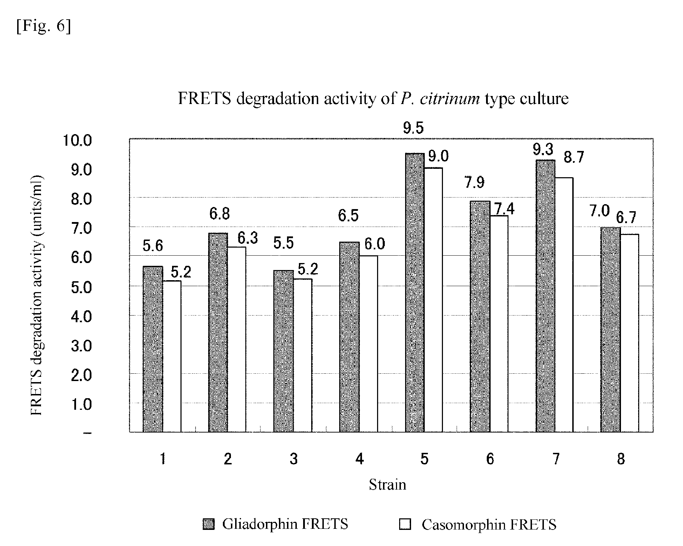

The measurement results are shown in Fig. 6. For all the strains, the culture supernatants favorably degraded gliadorphin and casomorphin. This result indicates that the enzyme (enzyme preparation) prepared from the culture supernatant of *Penicillium citrinum* is effective for the degradation of gliadorphin and casomorphin.

### 6. Evaluation of binding capacity of opioid peptide degradation product for opioid receptor

### (1) Method

The physiological activity of the gliadorphin degradation product and casomorphin degradation product was evaluated by Mu opioid receptor ligand binding assay kit (manufactured by Cisbio). This kit uses the cells which expressed the human Mu opioid receptor to determine the opioid receptor-bound molecules in the sample by the time resolved fluorescence (TR-FRET) method. The buffer was sodium acetate (pH 4.5) with the final concentration of 50 mM. The opioid peptides were gliadorphin, casomorphin, and a wheat-derived 33-mer peptide containing the opioid peptide sequence at the C-terminal side were individually added to the reaction system at the final concentration of 0.33 mg/ mL. The enzyme preparation from *Penicillium citrinum* (PCEP), the enzyme preparation from *Aspergillus oryzae* (AOEP), the enzyme preparation from *Aspergillus melleus* (AMEP), and papain were individually added at the final concentration of 50 mg/L. In consideration of the limit of detection of the kit, the test sections prepared using the above-described peptides and enzyme at a 10-fold concentration (10-fold concentrated sample) were also provided. After reaction for 120 minutes at 37°C, the enzyme was deactivated by boiling for 15 minutes, the solid content was removed from the supernatant by centrifugation at 15,000 rpm for 10 minutes, and the supernatant was used as the sample for evaluation using the kit.

### (2) Result

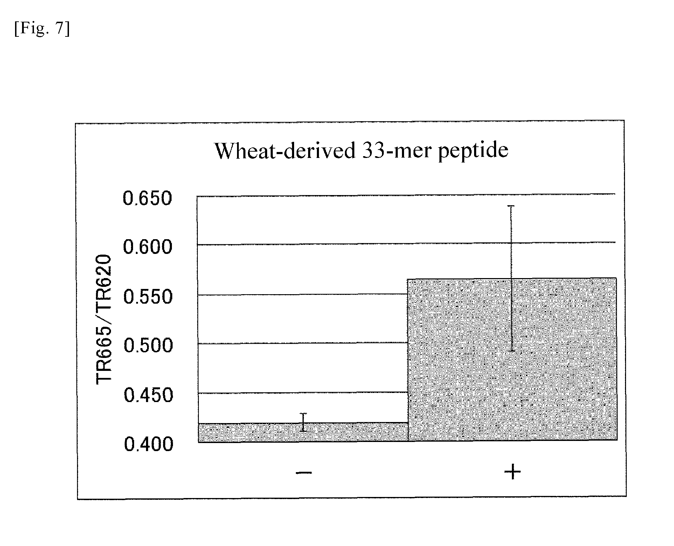

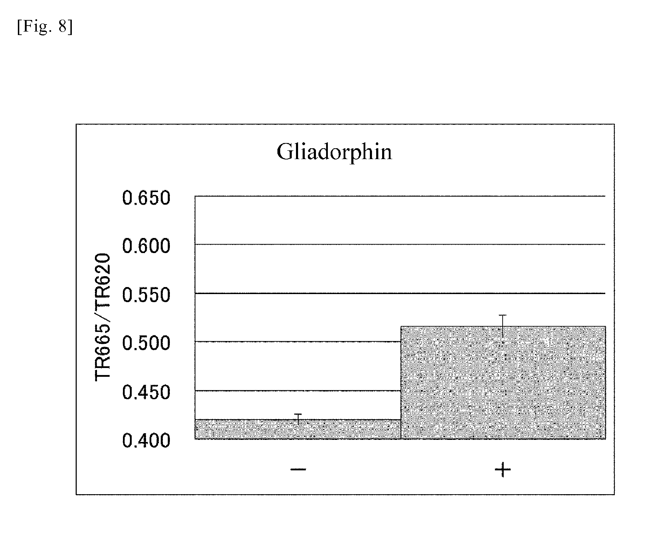

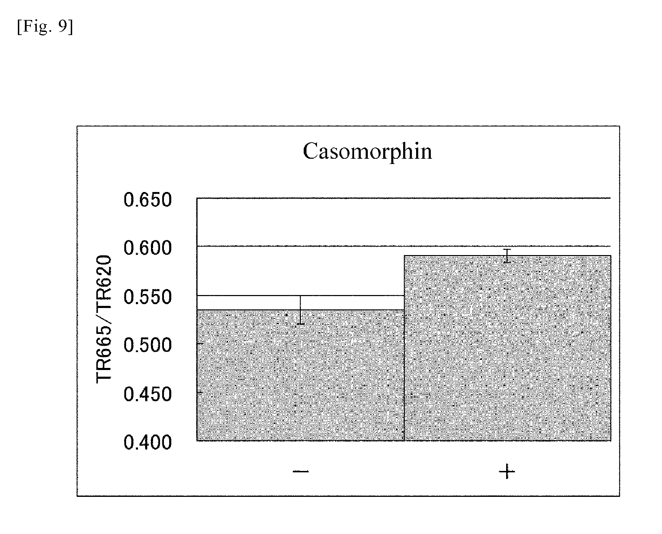

According to the protocol of the kit, the value of TR665 nm/TR620 nm was acquired and calculated. The results of each sample were shown in Figs. 7 to 9. According to this kit, a terbium-labeled opioid receptor binding ligand and an opioid receptor-bound molecule in the sample are competitively bound to the receptor, and the fluorescence is measured. Therefore, the higher the value of TR665 nm/TR620 nm is, the less opioid receptor-bound molecules are present in the sample, and the higher the value is, the more opioid receptor-bound molecule are present in the sample. For all the peptides, the TR665 nm/TR620 nm value was higher for those with enzyme addition (indicated with "+" in the figures) than those without enzyme addition (indicated with "-" in the figures). This result means that the number of the opioid receptor-bound molecules decreased by the enzyme addition.

As described above, three types of peptide, namely two opioid peptides and a wheat-derived 33-mer peptide containing an opioid peptide sequence on the side of the C-terminal, were degraded using an opioid degradation enzyme, and the degradation products were evaluated using the Mu opioid receptor ligand binding assay kit (manufactured by Cisbio); decrease in the receptor binding capacity was found in the degradation products. This result, the LC-MS result, and the result obtained using the casomorphin-gliadorphin determination kit suggest that the enzyme preparation degrades opioid peptides, and thus reduces the biological activity, or the binding capacity for the receptor.

### INDUSTRIAL APPLICABILITY

The enzyme preparation of the present invention exhibits a degradation activity for exogenous opioid peptides, and is expected to be used for the treatment of exogenous opioid peptide-related diseases such as autism.

The present invention will not be limited to the description of the embodiments and examples of the present invention. Various modifications readily made by those skilled in the art are also included in the present invention, without departing from the scope of claims. The entire contents of the articles, unexamined patent publications, and patent applications specified herein are hereby incorporated herein by reference.

## Claims

1. An exogenous opioid peptide-degrading enzyme preparation comprising one or more components selected from the group consisting of an enzyme preparation from *Penicillium citrinum,* an enzyme preparation from *Aspergillus oryzae,* and an enzyme preparation from *Aspergillus melleus,* wherein the exogenous opioid peptide-degrading enzyme preparation exhibits a degradation activity for a wheat gluten-derived opioid peptide and a casein-derived opioid peptide.

2. The exogenous opioid peptide-degrading enzyme preparation of claim 1, which comprises the above-described three enzyme preparations.

3. The exogenous opioid peptide-degrading enzyme preparation of claim 1 or 2, wherein the wheat gluten-derived opioid peptide is a gliadorphin comprising the amino acid sequence set forth in SEQ ID NO: 2, and the casein-derived opioid peptide is a casomorphin comprising the amino acid sequence set forth in SEQ ID NO: 3.

4. The exogenous opioid peptide-degrading enzyme preparation of any one of claims 1 to 3, wherein the enzyme preparation from *Aspergillus melleus* comprises a semi-alkaline protease.

5. A pharmaceutical or a food composition for treating exogenous opioid peptide-related diseases, comprising the exogenous opioid peptide-degrading enzyme preparation of any one of claims 1 to 4.

6. The composition of claim 5, wherein the exogenous opioid peptide-related disease is autism.

7. A therapy for an exogenous opioid peptide-related disease, comprising a step of administering the pharmaceutical composition of claim 6 in a therapeutically effective amount to a patient with an exogenous opioid peptide-related disease.

8. The therapy of claim 7, wherein the exogenous opioid peptide-related disease is autism.

9. A use of the exogenous opioid peptide-degrading enzyme preparation of any one of claims 1 to 4 for producing a pharmaceutical or food composition for treating an exogenous opioid peptide-related disease.

10. The use of claim 9, wherein the exogenous opioid peptide-related disease is autism.


SEQUENCE LISTING

<110>  AMANO ENZYME INC.
       MATSUBARA, Hirotaka
       IDO, Hiroki
       KOIKEDA, Satoshi

<120>  ENZYME PREPARATIONS USED FOR DIGESTION OF EXOGENOUS OPIOID
       PEPTIDES

<130>  AE11005P

<150>  JP P2011-215525
<151>  2011-09-29

<160>  8

<170>  PatentIn version 3.5

<210>  1
<211>  33
<212>  PRT
<213>  Triticum aestivum

<400>  1

Leu Gln Leu Gln Pro Phe Pro Gln Pro Gln Leu Pro Tyr Pro Gln Pro
1               5                   10                  15

Gln Leu Pro Tyr Pro Gln Pro Gln Leu Pro Tyr Pro Gln Pro Gln Pro
            20                  25                  30

Phe

<210>  2
<211>  7
<212>  PRT
<213>  Triticum aestivum

<400>  2

Tyr Pro Gln Pro Gln Pro Phe
1               5

<210>  3
<211>  7
<212>  PRT
<213>  Bos taurus

<400>  3

Tyr Pro Phe Pro Gly Pro Ile
1               5

<210>  4
<211>  5
<212>  PRT

<213>  Triticum aestivum

<400>  4

Gly Tyr Tyr Pro Thr
1               5


<210>  5
<211>  4
<212>  PRT
<213>  Triticum aestivum

<400>  5

Gly Phe Phe Pro
1


<210>  6
<211>  5
<212>  PRT
<213>  Triticum aestivum

<400>  6

Phe Gly Gly Tyr Leu
1               5


<210>  7
<211>  4
<212>  PRT
<213>  Triticum aestivum

<400>  7

Phe Gly Gly Tyr
1


<210>  8
<211>  7
<212>  PRT
<213>  Artificial Sequence

<220>
<223>  consensus sequence


<220>
<221>  misc_feature
<222>  (3)..(3)
<223>  Phe or Gln

<220>
<221>  misc_feature
<222>  (5)..(5)
<223>  Gly or Gln

<220>
<221>  misc_feature
<222>  (7)..(7)

<223>  Ile or Phe

<400>  8

Tyr Pro Xaa Pro Xaa Pro Xaa
1               5